# EUROPÄISCHE PATENTSCHRIFT

(11) **EP 1 136 093 B1**
(45) Veröffentlichungstag und Bekanntmachung des Hinweises auf die Patenterteilung: **24.08.2005**
(21) Anmeldenummer: 01106363.3
(22) Anmeldetag: 16.03.2001
(51) Int. Cl.: A61M 15/00

(54) **Vorrichtung zur kontrollierten Inhalation therapeutischer Aerosole**
Device for the controlled inhalation of a therapeutical aerosol
Dispositif pour l'inhalation controllée d'aérosol à usage médical

(30) Priorität: 17.03.2000 DE 10013093
(43) Veröffentlichungstag der Anmeldung: 26.09.2001
(73) Patentinhaber: InAMed GmbH, 35285 Gemünden (DE)
(72) Erfinder: Scheuch, Gerhard, Dr., 35285 Gemuenden (DE); Meyer, Thomas, Dr., 82343 Poecking (DE); Sommerer, Knut, 81241 München (DE); Greindl, Axel, Dr., 83626 Valley (DE); Müllinger, Bernhard, 84339 Unterdietfurt (DE)
(74) Vertreter: Vossius & Partner

(56) Entgegenhaltungen:
- EP-A- 1 159 020
- WO-A-92/07599
- WO-A-96/11723
- US-A- 5 363 842

## Beschreibung

Die Erfindung betrifft eine Vorrichtung zur kontrollierten Inhalation therapeutischer Aerosole und insbesondere die individuelle Dosimetrie von inhalierbaren Aerosolen.

Bei der Inhalation von Medikamenten in Aerosolform bestimmen mehrere Faktoren die Ablagerung des Wirkstoffes in der Lunge. Die Ablagerung des Wirkstoffes in der Lunge hängt ab von den Partikeleigenschaften des zu inhalierenden Wirkstoffes, wie etwa Teilchengröße, elektrische Ladung und Hygroskopizität, der Inhalationsgeschwindigkeit (d. h. Atemfluß) des Patienten und der Inhalationstiefe (d. h. Atemvolumen) eines Atemzuges des zu behandelnden Patienten.

Bei verschiedenen Medikamenten, die in Aerosolform inhalativ verabreicht werden sollen, muß die Menge des inhalierten Wirkstoffes genau dosiert werden, da eine eventuelle Überdosierung für den Patienten kritisch wäre. Bei herkömmlichen Inhalationsmethoden ist zwar die Partikelgröße an das zu verabreichende Medikament angepaßt, der Patient besitzt jedoch bezüglich seines Atemmusters eine große Freiheit, so daß die individuelle Dosierung sehr stark schwanken kann. Bei einem zu schwachen Einatemvorgang (flache, schnelle Einatmung) wird zu wenig Medikament verabreicht, während ein zu starkes Einatmen (tiefe, langsame Einatmung) eine Überdosierung zur Folge hat.

WO 92/07599 betrifft den Kalibrierungsprozess während der Herstellung eine Inhalationsgerätes von Typ NDI.

Die EP-B-0 587 380 beschreibt eine Arzneimittelzuführungsvorrichtung, die ein Einatmen erkennt und das Medikament jeweils nur während einer Inhalation appliziert, doch hat der Patient die Freiheit, beliebig zu atmen. Diese Freiheit schwankt überdies von Patient zu Patient, was zu einer erheblichen Variabilität in der Dosierung führt. Die EP-A-0 965 355 beschreibt einen gesteuerten Inhalator mit einem vorbestimmten Aerosolvolumen und einer Atemflußlimitierung. Bei diesem Inhalator werden Atemfluß und Atemvolumen in gewissen Grenzen eingestellt, doch ist dieser Inhalator als Massenprodukt nicht an die konkreten Voraussetzungen bezüglich Lungenfunktion eines einzelnen Individuums anzupassen. Für Atemvolumen und Atemfluß werden Parameter eingestellt, die für einen Großteil der Patienten akzeptabel sind, aber keine optimale Wirkstoffeinbringung für den einzelnen Patienten ermöglichen.

Deshalb treten in der Praxis die folgenden Probleme auf:
1. Viele schwer obstruktive Patienten können einen bestimmten erforderlichen Atemstrom nicht mehr aufbringen, den sie für eine optimale Aerosolapplikation aber erbringen müßten;
2. Viele dieser Patienten können nur noch sehr eingeschränkt Volumina inhalieren, was vor allem bei Patienten mit Lungenemphysem oder Patienten mit sehr kleinen Lungenvolumina der Fall ist;
3. Jeder Patient inhaliert mit unterschiedlicher Geschwindigkeit und Volumen, so daß es zu starken Schwankungen der in die Lunge gelangenden Medikamentendosis kommt.

Der Erfindung liegt die Aufgabe zugrunde, eine verbesserte Vorrichtung zur kontrollierten Inhalation therapeutischer Aerosole bereitzustellen. Diese Aufgabe wird mit den Merkmalen des unabhängigen Anspruchs gelöst.

Die Erfindung geht von dem Grundgedanken aus, bei einer Inhalationsvorrichtung eine Einrichtung vorzusehen, die individuelle Patientenparameter und/oder Aerosolparameter für die Inhalation bereitstellt, sowie eine Einrichtung vorzusehen, die die individuelle Dosierung des Aerosols bzw. der Aerosole abhängig von den bereitgestellten individuellen Patienten- und/oder Aerosolparametem vornimmt. Dadurch läßt sich erfindungsgemäß die Inhalationsvorrichtung individuell an die Möglichkeiten des Patienten adaptieren.

Gemäß einer ersten Ausführungsform werden die individuellen Parameter auf einem Speichermedium bereitgestellt, wie etwa auf Speichermedien erhältlich unter den Bezeichnungen SmartCard, FlashCard oder SmartLabel. Die individuellen Parameter werden beispielsweise nach einer Messung der aktuellen Lungenfunktion des Patienten (beispielsweise beim Hausarzt) in dem Speichermedium abgelegt. Dieses wird gemäß einer ersten Ausführungsform dann vom Patienten (zu Hause) in die Inhalationsvorrichtung eingesteckt, woraufhin die individuellen Daten ausgelesen werden. Alternativ wird das Speichermedium in eine separate Vorrichtung eingebracht, von der aus die individuellen Parameter an die Inhalationsvorrichtung übertragen werden. Gemäß einer weiteren alternativen Ausführungsform ist ein Modem vorgesehen, so daß der Inhalationsvorrichtung von dem behandelnden Arzt oder der betreuenden Institution die individuellen Parameter über eine Datenleitung (beispielsweise Telefonleitung) zugeführt werden.

Gemäß einer weiteren Ausführungsform ist eine Einrichtung für die manuelle Eingabe der individuellen Parameter vorgesehen, beispielsweise durch entsprechende Tasten. Alternativ werden bei der erfindungsgemäßen Inhalationsvorrichtung die individuellen Paramter über manuelle Regler bzw. manuelle Schalter eingestellt.

Die individuellen Patientenparameter und/oder Aerosolparameter wirken somit entweder manuell oder automatisch (beispielsweise über eine entsprechende Ventilsteuerung) auf die individuelle Dosierung des Aerosols bzw. der Aerosole ein. Da die Abhängigkeit der Deposition von Aerosol in bestimmten Bereichen der Lunge in Abhängigkeit von der Partikelgröße des Wirkstoffs, des Atemvolumens und des Atemflusses bekannt ist, kann somit erfindungsgemäß die Aerosoldeposition in der Lunge im wesentlichen voherbestimmt werden. Der Patient empfindet das eingestellte Atemmanöver als angenehm, da es an seine Möglichkeiten angepaßt ist.

Bevorzugt wird das jeweils vom Patienten durchgeführte Atemmanöver auf dem Speichermedium der Inhalationsvorrichtung gespeichert, so daß nach einer gewissen Therapiezeit eine Kontrolle der Anwendung durchgeführt werden kann.

Weiter bevorzugt ist das Speichermedium neu programmierbar, um bei Veränderungen der Lungenfunktion des Patienten entsprechend angepaßte Parameter für das richtige Atemmanöver bereitzustellen.

Bevorzugt wird mit der erfindungsgemäßen Inhalationsvorrichtung eine Überdosierung verhindert, beispielsweise durch Vorgeben einer Aktionszeit bzw. einer Aktionssperre, beispielsweise auf dem Speichermedium. Dies verhindert eine Aktivierung der Inhalationsvorrichtung durch den Patienten solange nicht die zwischen zwei aufeinanderfolgenden Inhalationen erforderliche Zeit verstrichen ist. Vorzugsweise dient das Speichermedium auch zur Fehlerprotokollierung. Beispielsweise wird protokolliert, ob eine zu hohe Abweichung des Verneblerdrucks von einem Soll-Bereich aufgetreten ist oder ob der erforderliche Vemeblerdruck überhaupt nicht aufgebaut werden konnte. Ferner wird vorzugsweise eine eventuell bei zu hohem Druck am Mundstück (Beatmungsdruck) auftretende Sicherheitsabschaltung auf dem Speichermedium protokolliert. Ferner bevorzugt ist die Protokollierung einer zu hohen Abweichung des Flusses (entweder Verneblerfluß des Aerosols oder Hilfsfluß der der Verneblerluft zugeführten Zusatzluft oder Summe der beiden Flüsse) oder einer Fehlermeldung, wenn einer der genannten Flüsse für die Inhalation nicht aufgebaut werden konnte. Vorzugsweise wird auch ein Abbruch der Inhalation vom Patienten protokolliert.

Vorzugsweise wird auf dem Speichermedium auch der Name des zu inhalierenden Medikaments gespeichert.

Ferner ist gemäß einer bevorzugten Ausführungsform eine Zugangsberechtigung für Service-Dienste bereitgestellt. Dabei wird über eine spezielle Kodierung des Speichermediums in der Inhalationsvorrichtung Software aktiviert, die für Servicezwecke zur Verfügung steht.

Die erfindungsgemäße Inhalationsvorrichtung weist die folgenden Vorteile auf:
1. Es wird für jeden Patienten ein individuell angenehmes und optimales Inhalationsmanöver eingestellt bzw. vorgegeben;
2. Durch die Vorgabe individueller Parameter können unterschiedliche Substanzen in verschiedene gewünschte Bereiche der Lunge appliziert werden;
3. Die Reproduzierbarkeit der Wirkstoffabgabe wird erhöht;
4. Dem Patienten wird die optimale Dosis des Wirkstoffs in den gewünschten Bereich der Lunge appliziert;
5. Durch die Programmierung von unterschiedlichen Atemmanövern können verschiedene Medikamente mit einem Gerät, jeweils optimal und an den Patienten angepaßt, inhaliert werden;
6. Die erfindungsgemäße Inhalationsvorrichtung kann umgehend auf neue Substanzen, neue Atemmanöver und geänderte Atemflüsse eingestellt werden (Update);
7. In einem Speichermedium, wie etwa einer SmartCard, können die Atemmanöver im Verlauf einer Therapie aufgezeichnet und anschließend ausgewertet werden;
8. Bei einer Veränderung der Lungenfunktion des Patienten kann die Inhalationsvorrichtung ohne weiteres auf die veränderten Rahmenbedingung eingestellt werden;
9. Die Verwendung eines Treibmittels ist nicht zwingend erforderlich.

Erfindungsgemäß können sämtliche Arzneistoffe verwendet werden, die entweder topisch im Respirationstrakt oder auch systemisch ihre Wirkung entfalten. Geeignete Arzneistoffe umfassen Analgetika, Antianginamittel, Antiallergica, Antihistamine und entzündungshemmende Mittel, Expectorantia, Antitussiva, Bronchodilatoren, Diuretika Anticholinergika, Corticoide, Xanthine, Antitumormittel sowie therapeutisch wirksame Proteine oder Peptide wie Insulin oder Interferon.

Bevorzugt ist die Verabreichung von Arzneistoffen zur Behandlung von respiratorischen Erkrankungen wie Asthma, sowie Mittel zur Prophylaxe und Behandlung der Schleimhäute des Tracheo-Bronchialtraktes. Besonders bevorzugt ist die Verabreichung von Estern des Retinols sowie der Retinsäure ("Vitamin A") wie in der EP-A-0 352 412 beschrieben. Die Arzneistoffe können in freier Form oder als pharmazeutisch verträgliches Salz oder als Ester vorliegen. Eine weitere Möglichkeit besteht darin, den Arzneistoff in Liposomen einzuschließen.

Die Arzneistoffe können mit üblichen pharmazeutisch verträglichen Excipienten konfektioniert werden.

## Patentansprüche

1. Vorrichtung zur kontrollierten Inhalation therapeutischer Aerosole mit einer Bereitstellungseinrichtung zum Bereitstellen von individuellen Patientenparametern und/oder Aerosolparametern für die Inhalation, und einer Einrichtung zum individuellen Dosieren der Aerosole abhängig von den bereitgestellten individuellen Patientenparametern und/oder Aerosolparametern, **dadurch gekennzeichnet daß** die Bereitstellungseinrichtung ein von einem Arzt, einer betreuenden Institution und/oder dem Patienten umprogrammierbares Speichermedium aufweist, in dem vor der Inhalation für jeden Patienten die für diesen Patienten individuellen Patientenparameter und/oder Aerosolparameter ablegbar sind.

2. Inhalationsvorrichtung nach Anspruch 1, wobei das Speichermedium ein aktives oder passives Speichermedium ist.

3. Inhalationsvorrichtung nach Anspruch 1 oder 2, wobei das Speichermedium ein FlashCard-, ein SmartCard- oder ein SmartLabel-Speichermedium ist.

4. Inhalationsvorrichtung nach Anspruch 1, wobei die Bereitstellungseinrichtung ferner ein Modem aufweist.

5. Inhalationsvorrichtung nach Anspruch 1, wobei die Bereitstellungseinrichtung ferner eine Eingabeeinrichtung für die manuelle Eingabe der individuellen Parameter aufweist.

6. Inhalationsvorrichtung nach Anspruch 1, 2 oder 3, wobei das Speichermedium die durchgeführten Atemmanöver speichert.

7. Inhalationsvorrichtung nach Anspruch 1, wobei die Bereitstellungseinrichtung ferner manuell betätigbare Regler und/oder Schalter aufweist.

8. Inhalationsvorrichtung nach einem der Ansprüche 1 bis 3, wobei die Einrichtung zum individuellen Dosieren der Aerosole die individuellen Patientenparameter und/oder Aerosolparameter für die Inhalation aus der Bereitstellungseinrichtung ausliest, auswertet und davon abhängig Atemfluß und Atemvolumen der Inhalationsvorrichtung einstellt.

9. Aufweisend Inhalationsvorrichtung nach einem der Ansprüche 1 bis 8, ferner Arzneistoffe, die topisch im Respirationstrakt oder auch systemisch ihre Wirkung entfalten.

## Claims

1. A device for the controlled inhalation of therapeutical aerosols, comprising means for providing individual patient parameters and/or aerosol parameters for the inhalation; and adjusting means for adjusting individual aerosol doses on the basis of the predetermined individual patient parameters and/or aerosol parameters, **characterised in that** said provision means comprise a memory medium which is re-programmable by a physician, a health care institution and/or the patient and in which the individual patient parameters and/or aeorsol parameters for each patient are storable before the inhalation.

2. The inhalation device according to claim 1, wherein the memory medium is an active or passive memory medium.

3. The inhalation device according to claim 1 or 2, wherein the memory medium is a FlashCard, SmartCard or SmartLabel memory medium.

4. The inhalation device according to claim 1, wherein the provision means further comprise a modem.

5. The inhalation device according to claim 1, wherein the provision means further comprise input means for manually inputting individual parameters.

6. The inhalation device according to claim 1, 2 or 3, wherein the memory medium stores the breathing manoeuvres carried out.

7. The inhalation device according to claim 1, wherein the provision means are further provided with manually operable control units and/or switches.

8. The inhalation device according to any of claims 1 to 3, wherein the adjusting means for adjusting the individual aerosol doses reads out the individual patient parameters and/or aerosol parameters for the inhalation from the provision means, evaluates them and, on the basis thereof, adjusts the respiratory flow and the tidal volume of the inhalation device.

9. The inhalation device according to any of claims 1 to 8 further comprising medicinal agents that become effective topically in the respiratory system or systemically.

## Revendications

1. Dispositif pour l'inhalation contrôlée d'aérosol à usage médical avec une installation de mise à disposition destinée à mettre à disposition des paramètres de patients individuels et/ou des paramètres d'aérosol pour l'inhalation, et une installation destinée au dosage individuel des aérosols en fonction des paramètres des patients individuels et/ou des paramètres des aérosols déterminés, **caractérisé en ce que** l'installation de mise à disposition présente un support de mémoire programmable par un médecin, un institut de soins et/ou le patient, dans lequel avant l'inhalation les paramètres de patients individuels et/ou les paramètres d'aérosol de ce patient peuvent être stockés en mémoire.

2. Dispositif d'inhalation selon la revendication 1, dans lequel le support de mémoire est un support de mémoire actif ou passif.

3. Dispositif d'inhalation selon la revendication 1 ou 2, dans lequel le support de mémoire est un support de mémoire à FlashCard, à SmartCard ou à SmartLabel.

4. Dispositif d'inhalation selon la revendication 1, dans lequel l'installation de mise à disposition présente en outre un modem.

5. Dispositif d'inhalation selon la revendication 1, dans lequel l'installation de mise à disposition présente en outre une installation d'introduction de données pour l'introduction manuelle des paramètres individuels.

6. Dispositif d'inhalation selon la revendication 1, 2 ou 3, dans lequel le support de mémoire enregistre les exercices de respiration réalisés.

7. Dispositif d'inhalation selon la revendication 1, dans lequel l'installation de mise à disposition présente en outre un régulateur et/ou un interrupteur actionnable manuellement.

8. Dispositif d'inhalation selon l'une des revendications 1 à 3, dans lequel l'installation destinée au dosage individuel des aérosols extrait les paramètres des patients individuels et/ou les paramètres d'aérosol pour l'inhalation de l'installation de mise à disposition, les exploite et en fonction définit le débit expiratoire et le volume inhalatoire du dispositif d'inhalation.

9. Dispositif d'inhalation selon l'une des revendications 1 à 8, comprenant en outre des médicaments, qui agissent de manière topique dans les voies respiratoires ou également de manière systémique.
